# EUROPEAN PATENT SPECIFICATION

(11) **EP 1 912 784 B1**
(45) Date of publication and mention of the grant of the patent: **07.10.2009**
(21) Application number: 06784981.0
(22) Date of filing: 15.06.2006
(51) Int. Cl.: B32B 7/02

(54) **MULTILAYER FILM**
MEHRSCHICHTFOLIE
FILM MULTICOUCHE

(30) Priority: 12.07.2005 US 595520 P; 22.11.2005 US 164447
(43) Date of publication of application: 23.04.2008
(73) Proprietor: BASF Catalysts LLC, Florham Park, NJ 07932 (US)
(72) Inventor: GRANEY, Daniel, LaGrangeville, NY 12540 (US); LEE, Eun, J., White Plains, NY 10603 (US); MILLER, Beth, Pompton Lakes, NJ 07442 (US); GIAMMATTEO, Marc, Wappingers Falls, NY 12590 (US)
(74) Representative: Huhn, Michael
(86) International application number: PCT/US2006/023455
(87) International publication number: WO 2007/008339

(56) References cited:
- WO-A-00/29212
- WO-A-93/15906
- WO-A-02/087875
- WO-A-20/05018931
- DATABASE WPI Week 200310 Derwent Publications Ltd., London, GB; AN 2003-111857 XP002399429 & WO 02/087875 A1 (SHISEIDO CO LTD) 7 November 2002 (2002-11-07)
- PATENT ABSTRACTS OF JAPAN vol. 2003, no. 09, 3 September 2003 (2003-09-03) & JP 2003 127303 A (SHISEIDO CO LTD), 8 May 2003 (2003-05-08)

## Description

### FIELD OF THE INVENTION

The present invention relates to multilayer co-extruded light-reflecting films which have a narrow reflection band because of light interference and contain a layer of polylactide.

### BACKGROUND OF THE INVENTION

Iridescent multilayer films are composed of a plurality of generally parallel layers of transparent thermoplastic resinous material in which the contiguous adjacent layers are of diverse resinous material whose index of refraction differs by at least about 0.03. The film contains at least 10 layers and more usually at least 35 layers and, preferably, at least about 70 layers.

The individual layers of the film are very thin, usually in the range of about 30 to 500 nm, preferably about 50-400 nm, which causes constructive interference in light waves reflected from the many interfaces. Depending on the layer thickness and the refractive index of the polymers, one dominant wavelength band is reflected and the remaining light is transmitted through the film. The reflected wavelength is determined by the sum of the optical thickness of a pair of layers.

The quantity of the reflected light (reflectance) and the color intensity depend on the differences between refractive indices, on the ratio of optical thicknesses of the layers, on the number of layers and on the uniformity of the thickness. If the refractive indices are the same, there is no reflection at all from the interfaces between the layers. In multilayer iridescent films, the refractive indices of contiguous adjacent layers differ by at least 0.03 and preferably by at least 0.06 or more. For first order reflections, reflectance is highest when the optical thicknesses of the layers are equal, although suitably high reflectances can be achieved when the ratio of the two optical thicknesses falls between 5:95 and 95:5. Distinct color reflections are obtained with as few as 10 layers. However, for maximum color intensity, it is desirable to have between 35 and 1000 or more layers. High color intensity is associated with a reflection band which is relatively narrow and which has high reflectance at its peak. It should be recognized that although the term "color intensity" has been used here for convenience, the same considerations apply for the invisible reflection in the ultraviolet and infrared ranges.

The multilayer films can be made by a chill-roll casting technique using a conventional single manifold flat film die in combination with a feedblock which collects the melts from each of two or more extruders and arranges then into the desired layer pattern. Feedblocks are described for instance in U.S. Pat. Nos. 3,565,985 and 3,773,882. The feedblocks can be used to form alternating layers of either two components or more (e.g. ABABAB ... , ABCABC ... or ACBCACBC ... ). The very narrow multilayer stream flows through a single manifold flat film die where the layers are simultaneously spread to the width of the die and thinned to the final die exit thickness. The number of layers and their thickness distribution can be changed by inserting a different feedblock module. Usually, the outermost layer or layers on each side of the sheet are thicker than the other layers. This thicker skin may consist of one of the components which makes up the optical core, may be a different polymer which is utilized to impart desirable mechanical, heat sealing, or other properties, or may be a combination of these.

Examination of iridescent films of desirable optical properties revealed deficiencies in certain mechanical properties. For example, the adhesion between individual layers of the multilayer structure may be insufficient, and the film may suffer from internal delamination or separation of layers during use. The iridescent film is often adhered to paper or board for its decorative effect, and is then used for greeting cards, cartons, wrapping paper and the like. Delamination of the film is unsightly and may even lead to separation of the glued joints if constructed as a carton. In addition, the solvent resistance and heat stability of such films are not as great as desired for widespread utilization.

In U.S. Pat. No. 4,310,584, these deficiencies are significantly overcome by using a thermoplastic terephthalate polyester or copolyester resin as the high refractive index component of the system in which two or more resinous material form a plurality of layers. While a substantial improvement was realized, it also required the use of two polymers from significantly different polymer families. That fact, in turn, means that there are inherent significant differences between the two polymers and their relative adhesion to each other, chemical resistance, toughness, etc. As a result, the film itself is generally no better with regard to a particular characteristic than the weaker or poorer of the polymers employed. If two polymers closely related were employed in order to maximize relative adhesion to one each other, or toughness, or chemical resistance, etc., the polymers involved did not have a sufficient difference in refractive index so as to create the desired iridescent color.

Schrenk and Wheatly (Co-extruded Elastomeric Optical Interference Film, Antec '88, 1703-1707) have reported the preparation of a multilayer light reflecting film co-extruded from two thermoplastic elastomers. The film which had one thermoplastic elastomer based on nylon and the other based on urethane, exhibited reversible changes in reflection spectra when deformed and relaxed. That is, this very specific combination had the ability of stretching without losing appearance characteristics. This type of films has been described in more detail in U.S. Pat. No. 4,937,134.

U.S. Pat. No. 5,089,318 discloses that further improvements in adhesion, solvent resistance and the like can be obtained by employing a thermoplastic elastomer (TPE) as one of the resinous materials. Such materials are copolymers of a thermoplastic hard segment such as polybutyl terephthalate, polyethylene terephthalate, polycarbonate, etc., and a soft elastomeric segment such as polyether glycols, silicone rubbers, polyetherimide and the like.

### SUMMARY OF THE INVENTION

The present invention provides another iridescent film that surprisingly provides significant improvements over current known structures with regard to these desirable properties. It is, therefore an aspect of the invention to provide a transparent thermoplastic resinous laminate having excellent adhesion and color properties, equal to or greater than prior art films.

The present invention provides a film comprising a plurality of contiguous layers that are substantially parallel to each other wherein at least every other of the contiguous layers is made of polylactide and the contiguous layers that are adjacent to the polylactide layers have a refractive index differing from the refractive index of the polylactide layers by at least about 0.03. This structure is an iridescent film. The term "polylactide" as used herein includes polylactic acid and all copolymers and blends of polylactide homopolymers and copolymers and is abbreviated as PLA.
In one embodiment, the present invention provides a transparent thermoplastic resinous laminate of at least 10 very thin layers of substantially uniform thickness, said layers being generally parallel, the contiguous adjacent layers being of different transparent thermoplastic resinous materials of which one is a poly(lactide) or poly(lactic acid) (PLA), the contiguous adjacent layers differing in refractive index by at least about 0.03. The poly(lactide) or poly(lactic acid) may be in at least one inner layer, multiple inner layers, all of every other of the inner layers, at least one outer layer, or both outer layers.

Other objects and advantages of the present invention will become apparent from the following description and appended claims.

### DETAILED DESCRIPTION OF THE INVENTION

The phrase "plurality of contiguous layers" as used herein means at least ten layers.

The phrase "substantially parallel layers" as used herein means that adjacent layers remain generally in the x-y plane and have minimal or no z direction shift.

The phrase "at least every other of said layers" as used herein includes every other layer, every third layer, every fourth layer, or other repeating structure. As examples if A represents one layer, B represents another layer and C represents another layer, a film of the present invention may comprise at least ten layers as ABABABABAB or ABCABCABCA.

The phrase "second thermoplastic polymer" as used herein means a polymer that has a different refractive index than the first thermoplastic polymer.

It has now been found that the objectives of this invention are realized by employing a biodegradable polymer poly(lactide) or poly(lactic acid) (PLA) resin as a component in the contiguous adjacent layers in the optical core of a transparent thermoplastic resinous laminate film. Polylactic acid belongs to a family of polymers referred to as aliphatic polyesters. Polylactic acid is a thermoplastic and is characterized by relatively high strength and modulus and by a low index of refraction of about 1.45. It is made from renewable resources and is compostable/biodegradable.

PLA is generally classified as a polyester because it has an ester group (C-CO-O-C) within its repeat unit. Specifically PLA is an aliphatic polyester with small methyl groups attached to the backbone. In comparison, polyethylene terephthalate (PET) is an aromatic polyester with a benzene ring incorporated into the backbone of each repeat unit. The rings of the aromatic polyester cause the PET polymer chain to remain linear, while PLA tends to form helical structures. Thus, PLA's structural makeup allows it to crystallize much more readily than PET.

PLA's polymer architecture can vary, and as a result, affect properties. The variation in architecture results from the different proportions of enantiomers of lactic acid, D(-) and L (+), that are used in the synthesis. These lactic acids can produce three types of lactides: D, L, and meso, for polymerization. PLA resins containing more than 93% of L-lactic acid units are semi-crystalline, while PLA with 50-93% content of L-lactic acid is strictly amorphous. The homopolymers poly(D-lactide) or poly(L-lactide) and high D- or L- copolymers have very regular structures and develop a crystalline phase. The presence of both meso and D-lactide forms produces imperfections in the crystalline structure, reducing the percent crystallinity. The T_{g} is also determined by the proportions of different lactides, around 71 degrees Celsius for 98% L lactide or 66 degrees Celsius for 94% L lactide. In comparison, the T_{g} for PET is around 80 degrees Celsius. The difference in chemical structure also contributes to the difference in other physical properties. PLA's surface energy of 42 dyne/cm is comparable to PET's at 43 dynes/cm, which provides for good printability. As previously mentioned, PLA has a refractive index of 1.45 which is similar to polymethylmethacrylate (PMMA). PET, on the other hand, has a refractive index of 1.57. Combining polylactic acid with higher refractive indexed polymers such as polyethylene terephthalate (PET) and polybutylene terephthalate (PBT) or lower refractive indexed polymers creates optical effects resulting in improved iridescent films. Polylactic acid is obtained, for example, from NatureWorks LLC, Minnetonka, Minn., U.S.A.

The registry number of PLA is 26680-10-4. The component registry Number is 95-96-5. The Formula: for PLA is C6 H8 04. The CA Index Name for PLA is 1,4-Dioxane-2,5-dione, 3,6-dimethyl-, homopolymer (9CI). Other names for PLA follow: p-Dioxane-2,5-dione, 3,6-dimethyl-, polyesters (8Cl); 3,6-Dimethyl-1,4-dioxane-2,5-dione homopolymer; DL-3,6-Dimethyl-1,4-dioxane-2,5-dione homopolymer; DL-Dilactide homopolymer; DL-Dilactide polymer; DL-Lactide homopolymer; DL-Lactide polymer; Lactide homopolymer; Lactide polymer; Poly(DL-lactide); Poly-dl-lactide; Polylactide; Poly(lactic acid).

The iridescent film of the present invention can be obtained by co-extruding the polylactic acid polyester or copolyester resin with a different transparent thermoplastic resin which is selected to differ in refractive index by at least 0.03 and preferably by at least 0.06. This difference in refractive index between adjacent layers results in an iridescent film when the film is exposed to light. Among the other resinous materials which can be used are transparent thermoplastic polyester or copolyester resins characterized by a refractive index of about 1.55 to about 1.61. Examples of usable thermoplastic polyester resins include terephthalate thermoplastic polyesters such as polyethylene terephthalate (PET) which is made by reacting either terephthalic acid or dimethyl terephthalate with ethylene glycol; polybutylene terephthalate (PBT) which is made by the catalyzed combination of 1,4-butanediol with either terephthalic acid or dimethyl terephthalate; and the various thermoplastic copolyesters which are synthesized using more than one glycol and/or more than one dibasic acid. Polyethylene terephthalate glycol (PETG) polyester, for example, is a glycol modified PET made from ethylene glycol and cyclohexanedimethanol (CHDM) and terephthalic acid; PCTA copolyester is an acid-modified copolyester of CHDM with terephthalic and isophthalic acids. Some additional other resinous materials that may be coextruded with the polylactic acid polyester or copolyester resin are listed in Table 1.

**TABLE 1**

| POLYMER NAME | APPROXIMATE REF INDEX |
|---|---|
| Poly(tetrafluoroethylene-co-hexafluoropropylene) | 1.338 |
| Poly(pentadecafluorooctyl acrylate) | 1.339 |
| Poly(tetrafluoro-3-(heptafluoropropoxy)propyl acrylate) | 1.346 |
| Poly(tetrafluoro-3-(pentafluoroethoxy)propyl acrylate | 1.348 |
| Poly(tetrafluoroethylene) | 1.35 (-1.38) |
| Poly(undecafluorohexyl acrylate) | 1.356 |
| Poly(nonafluoropentyl acrylate) | 1.360 |
| Poly(tetrafluoro-3-(trifluoromethoxy)propyl acrylate) | 1.360 |
| Poly(pentafluorovinyl propionate) | 1.364 |
| Poly(heptafluorobutyl acrylate) | 1.367 |
| Poly(trifluorovinyl acetate) | 1.375 |
| Poly(octafluoropentyl acrylate) | 1.380 |
| Poly(pentafluoropropyl acrylate) | 1.385 |
| Poly(2-(heptafluorobutoxy)ethyl acrylate) | 1.390 |
| Poly(2,2,3,4,4,4-hexafluorobutyl acrylate) | 1.392 |
| Poly(trifluoroethyl acrylate) | 1.407 |
| Poly(2-(1,1,2,2-tetrafluoroethoxy)ethyl acrylate) | 1.412 |
| Poly(trifluoroisopropyl methacrylate) | 1.4177 |
| Poly(2,2,2-trifluoro-1-methylethyl methacrylate) | 1.4185 |
| Poly(2-(trifluoroethyoxy)ethyl acrylate) | 1.419 |
| Poly(trifluorochloroethylene) | 1.42-1.43 |
| Poly(vinylidene fluoride) | 1.42 |
| Poly(dimethylsilylene(poly(dimethyl siloxane))) | 1.43 |
| Poly(trifluoroethyl methacrylate) | 1.437 |
| Poly(oxypropylene) | 1.4495 |
| Poly(vinylisobutyl ether) | 1.4507 |
| Poly(vinylethyl ether) | 1.4540 |
| Poly(oxyethylene) | 1.4563 |
| Poly(vinyl butyl ether) | 1.4563 |
| Poly(vinyl pentyl ether) | 1.4581 |
| Poly(vinyl hexy ether) | 1.4591 |
| Poly(4-methyl-1-pentene) | 1.459-1.465 |
| Cellulose acetate butyrate | 1.46-1.49 |
| Poly(4-fluoro-2-trifluoromethylstyrene) | 1.46 |
| Poly(vinyl octyl ether) | 1.4613 |
| Poly(vinyl 2-ethylhexyl ether) | 1.4626 |
| Poly(vinyl decyl ether) | 1.4628 |
| Poly(2-methoxyethyl acrylate) | 1.463 |
| Poly(butyl acrylate) | 1.4631 |
| Poly(butyl acrylate) | 1.466 |
| Poly(tert-butyl methacrylate) | 1.4638 |
| Poly(vinyl dodecyl ether) | 1.4640 |
| Poly(3-ethoxypropyl acrylate) | 1.465 |
| Poly(oxycarbonyl tetramethylene) | 1.465 |
| Poly(vinyl propionate) | 1.4665 |
| Poly(vinyl acetate) | 1.4665 |
| Poly(vinyl methyl ether) | 1.467 |
| Poly(ethyl acrylate) | 1.4685 |
| Poly(ethylene-co-vinyl acetate) | 1.47-1.50 |
| (30%-20% vinyl acetate) | |
| Cellulose proprionate | 1.47-1.49 |
| Cellulose acetate propionate | 1.47 |
| Benzyl cellulose | 1.47-1.58 |
| Phenol-formaldehyde resins | 1.47-1.70 |
| Cellulose triacetate | 1.47-1.48 |
| Poly(vinyl sec-butyl ether) (isotactic) | 1.4700 |
| Poly(3-methoxypropyl acrylate) | 1.471 |
| Poly(2-ethoxyethyl acrylate) | 1.471 |
| Poly(methyl acrylate) | 1.472-1.480 |
| Poly(isopropyl methacrylate) | 1.4728 |
| Poly(1-decene) | 1.4730 |
| Poly(propylene) (atactic, density 0.8575 g/cm.sup.3) | 1.4735 |
| Poly(vinyl sec-butyl ether)(isotactic) | 1.4740 |
| Poly(dodecyl methacrylate) | 1.4740 |
| Poly(oxyethyleneoxysuccinoyl) (poly(ethylene succinate)) | 1.4744 |
| Poly(teradecyl methacrylate) | 1.4746 |
| Poly(ethylene-co-propylene) (EPR-rubber) | 1.4748-1.48 |
| Poly(hexadecyl methacrylate) | 1.4750 |
| Poly(vinyl formate) | 1.4757 |
| Poly(2-fluoroethyl methacrylate) | 1.4768 |
| Poly(isobutyl methacrylate) | 1.477 |
| Ethyl cellulose | 1.479 |
| Poly(vinyl acetal) | 1.48-1.50 |
| Cellulose acetate | 1.48-1.50 |
| Cellulose tripropionate | 1.48-1.49 |
| Poly(oxymethylene) | 1.48 |
| Poly(vinyl butyral) | 1.48-1.49 |
| Poly(n-hexyl methacrylate) | 1.4813 |
| Poly(n-butyl methacrylate) | 1.483 |
| Poly(ethylidene dimethacrylate) | 1.4831 |
| Poly(2-ethoxyethyl methacrylate) | 1.4833 |
| Poly(oxyethyleneoxymaleoyl) (poly(ethylene maleate)) | 1.4840 |
| Poly(n-propyl methacrylate) | 1.484 |
| Poly(3,3,5-trimethylcyclohexyl methacrylate) | 1.485 |
| Poly(ethyl methacrylate) | 1.485 |
| Poly(2-nitro-2-methylpropyl methacrylate) | 1.4868 |
| Poly(triethylcarbinyl methacrylate) | |
| Poly(1,1-diethylpropyl methacrylate) | 1.4889 |
| Poly(methyl methacrylate) | 1.4893 |
| Poly(2-decyl-1,3-butadiene) | 1.4899 |
| Poly(vinyl alcohol) | 1.49-1.53 |
| Poly(ethyl glycolate methacrylate) | 1.4903 |
| Poly(3-methylcyclohexyl methacrylate) | 1.4947 |
| Poly(cyclohexyl-alpha-ethoxyacrylate) | 1.4969 |
| Methyl cellulose (low viscosity) | 1.497 |
| Poly(4-methylcyclohexyl methacrylate) | 1.4975 |
| Poly(decamethylene glycol dimethacrylate) | 1.4990 |
| Poly(urethanes) | 1.5-1.6 |
| Poly(1,2-butadiene) | 1.5000 |
| Poly(vinyl formal) | 1.50 |
| Poly(2-bromo-4-trifluoromethylstyrene) | 1.5 |
| Cellulose nitrate | 1.50-1.514 |
| Poly(sec-butyl.alpha.-chloroacrylate) | 1.500 |
| Poly(2-heptyl-1,3-butadiene) | 1.5000 |
| Poly(ethyl-alpha-chloroacrylate) | 1.502 |
| Poly(2-isopropyl-1,3-butadiene) | 1.5028 |
| Poly(2-methylcyclohexylmethacrylate) | 1.5028 |
| Poly(propylene) (density 0.9075 g/cm.sup.3) | 1.5030 |
| Poly(isobutene) | 1.505-1.5.1 |
| Poly(bornylmethacrylate) | 1.5059 |
| Poly(2-tert-butyl-1,3-butadiene) | 1.5060 |
| Poly(ethylene glycol dimethacrylate) | 1.5063 |
| Poly(cyclohexyl methacrylate) | 1.5066 |
| Poly(cyclohexanediol-1,4-dimethacrylate) | 1.5067 |
| Butyl rubber (unvulcanized) | 1.508 |
| Poly(tetrahydrofurfuryl methacrylate) | 1.5096 |
| Gutta percha (5) | 1.509 |
| Poly(ethylene) ionomer | 1.51 |
| | 1.51-1.54 |
| poly(oxyethylene) (high molecular weight) | |
| Poly(ethylene) (density 0.914 g/cm.sup.3) | 1.51 |
| (density 0.94-0.945 g/cm.sup.3) | 1.52-1.53 |
| (density 0.965 g/cm.sup.3) | 1.545 |
| Poly(1-methylcyclohexyl methacrylate) | 1.5111 |
| Poly(2-hydroxyethyl methacrylate) | 1.5119 |
| Poly(vinyl chloroacetate) | 1.512 |
| Poly(butane)(isotactic) | 1.5125 |
| Poly(vinyl methacrylate) | 1.5129 |
| Poly(N-butyl-methacrylamide) | 1.5135 |
| Gutha percha (.alpha.) | 1.514 |
| Terpene resin | 1.515 |
| Poly(1,3-butadiene) | 1.5154 |
| Shellac | 1.51-1.53 |
| Poly(methyl.alpha.-chloroacrylate) | 1.517 |
| Poly(2-chloroethyl methacrylate) | 1.517 |
| Poly(2-diethylaminoethyl methacrylate) | 1.5174 |
| Poly(2-chlorocyclohexyl methacrylate) | 1.5179 |
| Poly(1,3-butadiene) (35% cis; 56% trans; 7% 1,2 content) | 1.5180 |
| Natural rubber | 1.519-1.52 |
| Poly(allyl methacrylate) | 1.5196 |
| Poly(vinyl chloride) + 40% dioctyl phthalate | 1.52 |
| Poly(acrylonitrile) | 1.52 |
| | 1.5187 |
| Poly(methacrylonitrile) | 1.52 |
| Poly(1,3-butadiene) (high cis-type) | 1.52 |
| Poly(butadiene-co-acrylonitrile) | 1.52 |
| Poly(methyl isopropenyl icetone) | 1.5200 |
| Poly(isoprene) | 1.521 |
| Poly(ester) resin, rigid (ca, 50% styrene) | 1.523-1.54 |
| Poly(N-(2-methoxyethyl)methacrylamide) | 1.5246 |
| Poly(2,3-dimethylbutadiene) (methyl rubber) | 1.535 |
| Poly(vinyl chloride-co-vinyl acetate) (95/5-90/10) | 1.525-1.536 |
| Poly(acrylic acid) | 1.527 |
| Poly(1,3-dichioropropyl methacrylate) | 1.5270 |
| Poly(2-chloro-1-(chloromethyl)ethyl methacrylate) | 1.5270 |
| Poly(acrolein) | 1.529 |
| Poly(1-vinyl-2-pyrrolidone) | 1.53 |
| Hydrochlorinated rubber | 1.53-1.55 |
| Nylon 6: Nylon 6,6: Nylon 6, 10 (moulding) | 1.53 |
| (Nylon-6-fiber: 1.515 transverse. 1/565 in fiber direction) | |
| Poly(butadiene-co-styrene) (ca,30% styrene) black copolymer | 1.53 |
| Poly(cyclohexyl .alpha.-chloroacrylate) | 1.532 |
| Poly(butadiene-co-styrene) (ca, 75/25) | 1.535 |
| Poly(2-aminoethyl methacrylate) | 1.537 |
| Poly(furfuryl methacrylate) | 1.5381 |
| Proteins | 1.539-1.541 |
| Poly(1-phenyl-n-amyl methacrylate) | 1.5390 |
| Poly(N-methyl-methacrylamide) | 1.5398 |
| Cellulose | 1.54 |
| Poly(vinyl chloride) | 1.54-1.55 |
| Urea formaldehyde resin | 1.54-1.56 |
| Poly(sec-butyl-alpha-bromoacrylate) | 1.542 |
| Poly(cyclohexyl-alpha-bromoacrylate) | 1.542 |
| Poly(2-bromoethyl methacrylate) | 1.5426 |
| Poly(dihydroabietic acid) | 1.544 |
| Poly(abietic acid) | 1.546 |
| Poly(ethylmercaptyl methacrylate) | 1.547 |
| Poly(N-allyl methacrylamide) | 1.5476 |
| Poly(1-phenylethyl methacrylate) | 1.5487 |
| Poly(vinylfuran) | 1.55 |
| Poly(2-vinyltetrahydrofuran) | 1.55 |
| Poly(vinyl chloride) + 40% trictesyl phosphate | 1.55 |
| Epoxy resins | 1.55-1.60 |
| Poly(p-methoxybenyl methacrylate) | 1.552 |
| Poly(isopropyl methacrylate) | 1.552 |
| Poly(p-isopropylstyrene) | 1.554 |
| Poly(chloroprene) | 1.554-1.558 |
| Poly(oxyethylene)-alpha-benzoate-omega-methacrylate) | 1.555 |
| Poly(p,p'-xylylenyl dimethacrylate) | 1.5559 |
| Poly(1-phenylallyl methacrylate) | 1.5573 |
| Poly(p-cyclohexylphenyl methacrylate) | 1.5575 |
| Poly(2-phenylethyl methacrylate) | 1.5592 |
| | 1.5602 |
| Poly(oxycarbonyloxy-1,4-phenylene-1-propyl) butylidene-1,4 phenylene | |
| Poly(oxycarbonyloxy-1,4-phenylene-1-propyl) | 1.5624 |
| Poly(styrene-co-maleic anhydride) | 1.564 |
| Poly(1-phenylcyclohexyl methacrylate) | 1.5645 |
| Poly(oxycarbonyloxy-1,4-phenylene-1,3-dimethyl-butylidene-1,4-phenylene) | 1.5671 |
| Poly(methyl-alpha-bromoacrylate) | 1.5672 |
| Poly(benzyl methacrylate) | 1.5680 |
| Poly(2-phenylsulfonyl)ethyl methacrylate)poly(m-cresyl methacrylate) | 1.5682 |
| Poly(styrene-co-acrylonitrile) (ca, 75/25) | 1.57 |
| Poly(oxycarbonyloxy-1,4-phenyleleneisobutylidene-1,4-phenylene) | 1.5702 |
| Poly(o-methoxyphenyl methacrylate) | 1.5705 |
| Poly(phenyl methacrylate) | 1.5706 |
| Poly(o-cresyl methacrylate) | 1.5707 |
| Poly(diallyl phthalate) | 1.572 |
| Poly(2,3 -dibromopropyl methacryate) | 1.5739 |
| Poly(oxycarbonyloxy-1,4-phenylene-1-methyl-butylidene-1,4-phenylene) | 1.5745 |
| Poly(oxy-2,6-dimethylphenylene) | 1.575 |
| Poly(oxyethyleneoxyterephthaloyl) | 1.5750 |
| (amorphous)(poly(ethylene terephthalate)) (crystalline | |
| fiber: 1.51 transverse; 1.64 in fiber direction) | |
| Poly(vinyl benzoate) | 1.5775 |
| poly(oxycarbonyloxy-1,4-phenylenebutylidene-1,4-phenylene) | 1.5792 |
| Poly(1,2-diphenylethyl methacrylate) | 1.5816 |
| Poly(o-chlorobenzyl methacrylate) | 1.5823 |
| Poly(oxycarbonyloxy-1,4-phenylene-sec-butylidene-1,4-phenylene) | 1.5827 |
| Poly(oxypentaerythritoloxyphthaloyl) | 1.584 |
| Poly(m-nitrobenyl methacrylate) | 1.5845 |
| Poly(oxycarbonyloxy-1,4-phenyleneisopropylidene-1,4-phenylene) | 1.5845 |
| Poly(N-2-phenylethyl)methacrylamide) | 1.5857 |
| Poly(4-methoxy-2-methylstyrene) | 1.5868 |
| Poly(o-methylstyrene) | 1.5874 |
| Poly(styrene) | 1.59-1.592 |
| Poly(oxycarbonyloxy-1,4-phenylenecyclohexylidene-1,4-phenylene) | 1.5900 |
| Poly(o-methoxystyrene) | 1.5832 |
| Poly(diphenylmethyl methacrylate) | 1.5933 |
| Poly(oxycarbonyloxy-1,4-phenyleneethylidene-1,4-phenylene) | 1.5937 |
| Poly(p-bromophenyl methacrylate) | 1.5964 |
| Poly(N-benzyl methacrylamide) | 1.5965 |
| Poly(p-methoxystyrene) | 1.5967 |
| Hard rubber (32%S) | 1.6 |
| Poly(vinylidene chloride) | 1.60-1.63 |
| Poly(sulfides ("Thiokol")) | 1.6-1.7 |
| Poly(o-chlorodiphenylmethyl methacrylate) | 1.6040 |
| Poly(oxycarbonyloxy-1,4-(2,6-dichloro)phenylene-isopropylidene-1,4-(2,6-dichloro)phenylene) | 1.6056 |
| Poly(oxycarbonyloxybis(1,4-(3,5-dichlorophenylene)) . | 1.6056 |
| Poly(pentachiorophenyl methacrylate) | 1.608 |
| Poly(o-chlorostyrene) | 1.6098 |
| Poly(phenyl-alpha-bromoacrylate) | 1.612 |
| Poly(p-divinylbenzene) | 1.6150 |
| Polymethylmethacrylate (PMMA) | 1.49 |
| Polyethylene (LLDPE) | 1.50 |
| Polypropylene (PP) | 1.50 |
| Ethylene-Vinyl Acetate (EVA) | 1.49 |
| Poly(ethylene terephthalate) (PET) | 1.57 |
| Poly(ethylene naphthalate) (PEN) | 1.64 |

The iridescent film of the present invention can also be obtained by co-extruding the polylactic acid polyester or co-polyester resin with a polymer having a higher refractive index than the polylactic acid resin which is selected to differ in refractive index by at least about 0.03 and preferably at least 0.06.

The outermost layers of the iridescent film of the present invention can be the same or different from resins in the optical core. For example, the outermost layers can comprise a polyester or copolyester resin such as polybutylene terephthalate polyester or glycol modified polyethylene terephthalate like PETG polyester.

The number of layers in the iridescent film of the invention is at least 10 layers, preferably at least 35 layers and more preferably at least about 70 layers.

The individual layers of the iridescent film are very thin, usually in the range of generally about 15 nanometers (nm) to about 500 nm and preferably about 50 nm to about 400 nm. Generally, the thickness of the outer layers ranges from about 1 to about 8 microns.

One combination in accordance with this invention involves an iridescent film having the contiguous adjacent layers in the optical core being of different transparent thermoplastic resinous materials of which one is polylactic acid polyester or copolyester, and the other is a terephthalate thermoplastic polyester such as a polyethylene terephthalate polyester or copolyester or polybutylene terephthalate polyester or copolyester, wherein the outermost layers are polybutylene terephthalate or PETG polyester.

One skilled in the art can adjust the thickness of each layer and the number of layers to achieve films useful outside of the visible region such as IR or UV.

The use of polylactide in the present film provides a high tensile strength, certain barrier properties such as grease and oil and flavor aroma, compostability, good optics (gloss and clarity).

The film of this invention which contains the PLA can also be oriented either uniaxially or biaxially to increase strength and orient the crystals as known in the art. Importantly, it has been found that the refractive index of the PLA film is not extensively changed upon orientation. This is contrary to the effect that orientation has on aromatic polyesters such as PET in which the refractive index is increased upon orientation. Accordingly, orienting a PLA/PET film can vastly improve the color intensity since the difference in the refractive indexes of the respective film becomes greater upon orientation. The examples below will show that the greater the difference of refractive indexes between layers results in the exhibition of a more intense color.

The multilayer films are usually made by a chill-roll casting technique in which melts of the thermoplastic resinous material from two or more extruders are collected by a feedblock which arranges them into a desired layered pattern. The very narrow multilayer stream flows through a single manifold flat film die with the layers simultaneously spread to the width of the die and thinned to the final die exit thickness. The number of layers and their thickness distribution can be changed by using a different feedblock module. Usually, the outermost layer or layers on each side of the sheet is thicker than the internal layers so as to form a relatively thick skin in a substantially equal division. Preferably, the present film is made by a process disclosed in US Patent 3,801,429, incorporated herein by reference to the extent necessary to complete this disclosure.

Manufacturing films with biodegradable polylactic acid layers is environmentally desirable. By varying the proportion of PLA in the microlayers, the degree and rate of degradation may be controlled. If PLA is the major component, a faster decomposition can be expected. If the thickness of the individual layers is designed to be in a nanolayer region, the multiplayer films will have associated iridescent/optical effects. Pairs of polymers may be arranged in ratios from 50/50 to 90/10 depending on the desired optical effect and the properties of the individual polymers, though this is not a required qualification for a rate-controlling layered-design. Controlled rate degradation can also be accomplished via blending various polymers together in proportions designed to influence decomposition.

The applications for PLA containing films include packaging such as labels, decorative bags, shrink films, textile films, products fibers, threads etc. One of the biggest markets for iridescent film is decorative glitter (size-reduced from film). Incorporating polylactic acid into the film structure imparts some measure of degradability to the tiny particles that are often difficult to contain after use. Thus polylactic acid containing glitter is environmentally advantageous over nonpolylactic acid containing glitter.

The present invention may be used in flexible and rigid decorative packaging. Flexible decorative packaging includes but is not limited to wrapping paper, ribbons, and bows. Rigid decorative packaging includes but is not limited to cosmetic and personal care containers such as for skin care products such as facial mask, UV protective lotion, liquid soap, and antimicrobial product; hair care products such as shampoo, conditioner, hair spray or fixative, and hair colorant; makeup products such as nail polish, mascara, eye shadow, and perfume; shaving cream, deodorant, baby oil, and dental products. The present film may also be used in printed and laminated board for use in packaging. The present invention may also be used in graphic applications such as book covers. The present film may also be used in vertical form and fill packaging which is a type of packaging equipment which feed the packaging film into a shaped area where it can be heat sealed in any of several ways and the package is then filled with something and sealed shut. The dimensions of the finished package are determined by the width of the film fed into the machine and the length of the bag is controlled by the speed and frequency settings at the sealing head. Numerous items may be packaged into a finished iridescent film pouch or bag in this way. The present film may also be used in fashion accessories such as sequins and threads. The present film may also be used in picture frame profile wrapping. The present film may also be used in medical related applications.

Also, the present invention may be sized reduced in some manner to form glitter particles. These particles are of various size and shape depending on the application. The size ranges from very small, approximately .004", to larger particles.

The present film may also be used as a label for various containers. Such containers include but are not limited to cosmetic and personal care containers such as for skin care products such as facial mask, UV protective lotion, liquid soap, and antimicrobial product; hair care products such as shampoo, conditioner, hair spray or fixative, and hair colorant; makeup products such as nail polish, mascara, eye shadow, and perfume; shaving cream, deodorant, and baby oil.

Several properties of these films are important and can be tested. For example delamination resistance of a film is tested by restraining one surface of the film with adhesive tape. A second piece of adhesive tape is applied to the other surface of the film. This second piece of tape is then pulled away and any indications of delamination is noted. If no delamination is observed, the tape is reapplied and the test repeated until failure is noted. Different tapes with different tack levels can be used to more fully differentiate between various film structures. Additionally, the film sample being tested can be immersed in solvent prior to testing or may be scored to provide a more severe form of this test. The number of pulls to failure using a particular tape is typically recorded. A description of this test method can be found in U.S. Pat. No. 5,089,318.

To test the solvent resistance of the film, samples of the film are immersed in the challenge solvent. The sample is observed for any color change, for the time at which the solvent begins to affect the iridescent color of the film and the nature of the color change. The time to initial color change and the nature of the color change at set time intervals are recorded. This test is typically run for a period of seven days with observations taken throughout the seven-day period. At the end of the twenty-four hour period, the film sample is removed from the challenge solvent and allowed to dry for twenty-four hours. After the twenty-four hour drying period, the tester notes how the iridescent film color has changed. This data is referred to as the "Color Recovery".

As mentioned above, weak adhesion between individual layers of non-PLA containing films affects the potential possibilities of creating products with such films. Weak interlayer adhesion causes films to suffer internal delamination or separation of layers during use and thus prevents lamination of the final film product. Interlayer adhesion of a PET/PLA interface, of IF (1) and IF (2) described below, is greater than the more typical PET/PMMA interface as seen in CIF (1) or the PEN/PMMA interface of CIF (2). Adequate interlayer adhesion is a desirable and essential property for most film that is subsequently laminated or applied to another substrate.

To test the heat shrinkage of the film, a 2"x 2" piece of film is cut from the film in question. The color of this piece is measured using a spectrophotometer. Data measured include the dominant wavelength (DWL), peak wavelength (PWL), and % peak reflection of the sample. The test specimen is then placed in an oven at the test temperature for a period of fifteen minutes. The sample is then removed from the oven. The sample is measured using a ruler to determine the percentage of shrinkage experienced by the film. The color of the sample is re-measured and the changes in shrinkage, DWL, PWL and peak reflection are recorded.

### EXAMPLE 1- Iridescent Films IF(1)

Polylactide (NatureWorks product 4042D having 4-5% D lactide) was fed to a feedblock from one extruder and polyethylene terephthalate (PET) polyester or copolyester from a second extruder to form films with 226 layer. One film had an average thickness of 1.35 mil. Another film had an average thickness of 4.45 mil. Each film had an exterior skin of polyethylene terephthalate glycol mixture.

The resulting iridescent films of 1.35 mil in thickness displayed intense color effects, were brightly iridescent and shared the same properties. The films had a color measurement peak of 88.3%. The average yield tensile strength was 6941.2 psi and the average ultimate tensile was 6875.7 psi. The resulting film of 4.45 mil in thickness was oriented uniaxially in both transverse and machine direction to increase strength and orient the crystals as known in the art. The increase in refractive index on polyethylene terephthalate upon orientation in the machine direction increased the color intensity up to peak of 97%.

### EXAMPLE 2 - Iridescent Films IF(2)

Polylactide (NatureWorks product 4042D having 4-5% D lactide) was fed to a feedblock from one extruder and polyethylene terephthalate glycol (PETG) polyester or copolyester from a second extruder to form films with 226 layers. One film had an average thickness of 1.50 mil. Another film had an average thickness of 4.70 mil. Each film had an exterior skin of polyethylene terephthalate glycol mixture.

The resulting iridescent films of 1.50 mil in thickness displayed intense color effects, were brightly iridescent and shared the same properties. The films had a color measurement peak of 88.5%. The yield tensile strength is 6868.7 psi and the ultimate tensile is 8121.1 psi. The resulting film of 4.70 mil in thickness was oriented uniaxially in transverse direction to be applied as iridescent shrink film.

### EXAMPLES 3 and 4- Commercial Iridescent Films CIF(1) and CIF(2)

To compare the color properties of the films IF (1) and IF (2), tests were conducted on comparative film CIF(1) and comparative film CIF(2). CIF (1) comprised polyethylene terephthalate polyester and polymethyl methacrylate layers (PET/PMMA), while CIF (2) comprised polyethylene naphthalate polyester and polymethyl methacrylate layers (PEN/PMMA). The CIF (1) had an exterior skin of polyethylene terephthalate mixture, a color measurement peak of 76.6% and an average thickness of 1.31mil. The yield tensile strength was 6875.5 psi and the ultimate tensile was 6744.3 psi. The CIF (2) had an exterior skin of polyethylene terephthalate glycol additive, a color measurement peak of 95.7% and an average thickness of 1.35 mil.

The invention has been described above, but is more broadly applicable as will be understood by those skilled in the art. The scope of the invention is therefore limited only by the following claims.

### EXAMPLE 5

The adhesion test described above was conducted on both a commercial multi-layer film comprising PET/PMMA and a film of this invention comprising PET/PLA. What was found was that the PET/PMMA film delaminated during the test whereas no delamination was found with the inventive PET/PLA film. This improved internal bonding is important inasmuch as it indicates the inventive films can be laminated to other surfaces effectively.

### EXAMPLE 6 - Iridescent Films IF (3)

Polyactide (NatureWorks product 4042D having 4-5 percent D lactide) was fed to a feedblock from one extruder and polyethylene terephthalate (Eastman 9921 PET) from a second extruder to form films with 113 layers. The films had an average thickness of 0.78 mils. Each film had an exterior skin or skins of polybutylene terephthalate (GE Valox 315 PBT). The films displayed intense color effects, were brightly iridescent. The films had a color measurement peak of 80%.

### EXAMPLE 7-Iridescent Films IF (4)

Example 6 was repeated except that the exterior layers or skins were polylactide (NatureWorks product 4042D having 4-5% D lactide). The films displayed intense color effects, were brightly iridescent. The films had a color measurement peak of 80%.

### EXAMPLE 8

To test the ability to resist disintegration of optical and mechanical properties due to exposure to light, heat and water, samples of the film were exposed to simulated weather conditions via Xenon-Arc exposure inside an enclosed weathering chamber. A 5"x7" piece of film is cut from the film in question. The film is then immobilized in a clamp with the surface of the film exposed. The entire contraption then is placed inside a Xenon-Arc weathering chamber to be exposed to controlled irradiance and water-cooling. The test cycle is designed to accelerate extreme environmental conditions encountered such as sunlight, heat and moisture (in the form of humidity, condensation, or rain).

To compare the retention of tensile strength of iridescent films IF (3) and IF (4), the test described above was conducted on both a commercial multi-layer film comprising of PET/PMMA and a film of this invention comprising PET/PLA with exterior skin or skins of polybutylene terephthalate or Polylactide. The retention period of iridescent films IF (3) and IF (4) was twice as long as commercial films. The increase in this period indicates the inventive films will have longer durability upon exposure to outdoor conditions.

### EXAMPLE 9- Iridescent Films IF (5)

Polylactide (NatureWorks product 4042D having 4-5 percent D lactide) was fed to a feedblock from one extruder and polyethylene terephthalate from a second extruder to form films with 226 layers. The films had an average thickness of 1.40 mil and 4.10 mil. The films with thickness of 1.40 mil displayed intense color effects and were brightly iridescent. The films had a color measurement peak of 91 percent. The films with thickness of 4.10 mil may be oriented either uniaxially or biaxially to increase strength and orient the crystals as known in the art.

### EXAMPLE 10 - Iridescent Films IF (6)

Polyactide (NatureWorks product 4042D having 4-5 percent D lactide) was fed to a feedblock from one extruder and polyethylene terephthalate from a second extruder to form films with 226 layers. IF (6) is different from IF (5) with PLA being the major component, where a faster decomposition may be expected as described above.

### EXAMPLE 11 - Iridescent Films IF (7)

Polylactide (NatureWorks product 4042D having 4-5 percent D lactide) was fed to a feedblock from one extruder and polybutylene terephthalate from a second extruder to form films with 113 layers. Each film had an exterior skin or skins of polybutylene terephthalate. The films had an average thickness of 0.70 mil. The films displayed intense color effects and were brightly iridescent. The films had a color measurement peak of 81 percent. PLA was the major component. The average ultimate tensile was 7100 psi.

### EXAMPLE 12 - Iridescent Films IF (8)

Example 11 was repeated except that the major component was polybutylene terephthalate, which would contribute to higher tensile strength and solvent resistance. The films had an average thickness of 0.72 mil and a color measurement peak of 81 percent. The average ultimate tensile was 16,400 psi. The average percent elongation was 380.

### EXAMPLE 13 - Iridescent Films IF (9)

Example 12 was repeated except that Polylactide (NatureWorks product 4032D having 1-2 percent D lactide) was used. Using polylactide with a lower D lactide can contribute to higher heat resistance due to increased crystallinvity. The films had an average thickness of 0.72 mil and color measurement peak of 81 percent. The average ultimate tensile was 15,100 psi. The average percent elongation was 406.

### EXAMPLE 14 - Iridescent Films IF (10)

Example 12 was repeated except that exterior layers or skins were polyethylene terephthalate (PET) polyester or co-polyester. The films had an average thickness of 0.73 mil and color measurement peak of 79 percent. The average ultimate tensile was 12,400 psi. The average percent elongation was 420.

### EXAMPLE 15 - Iridescent Films IF (11)

Polylactide (NatureWorks product 4042D having 4-5% D lactide) was fed to a feedblock from one extruder and polyethylene terephthalate (PET) polyester or co-polyester from a second extruder to form films with 226 layers. Each film had an exterior skin of polymethyl methacrylate (PMMA) and butyl acrylate mixture. The films had an average thickness of 1.45 mil. The resulting iridescent films displayed intense color effects and were brightly iridescent. The films had a color measurement peak of 93-95 percent.

## Claims

1. A film comprising a plurality of contiguous layers that are substantially parallel to each other wherein at least every other of said contiguous layers is made of polyactide and said contiguous layers that are adjacent to said polylactide layers have a refractive index differing from the refractive of said polylactide layers by at least 0.03.

2. The film according to claim 1 wherein said contiguous layers that are adjacent to said polylactide layers are of a polyester different from said polylactide.

3. The film according to claim 1 or 2 wherein said contiguous layers that are adjacent to said polylactide layers are of a polyester having a higher refractive index than the refractive index of said polylactide.

4. The film according to any of claims 1 to 3 wherein said contiguous layers that are adjacent to said polylactide layers are of terephthalate polyester.

5. The film according to claim 4 wherein said terephthalate polyester is a polyethylene terephthalate glycol.

6. The film according to claim 4 wherein said terephthalate polyester is a polyethylene terephthalate.

7. The film according to claim 4 wherein the terephthalate polyester is a polyethylene terephthalate.

8. The film according to any of claims 1 to 7 having at least 35 layers.

9. The film according to any of claims 1 to 8 wherein said polylactide comprises more than 93 % of L-lactic acid units.

10. The film according to any of claims 1 to 9 wherein said polylactide comprises homopolymers poly(D-lactide) or poly(L-lactide) and high D- or L-copolymers.

## Patentansprüche

1. Folie mit mehreren aneinandergrenzenden Schichten, die zueinander weitgehend parallel sind, worin mindestens jede zweite der aneinandergrenzenden Schichten aus Polylactid besteht und die aneinandergrenzenden Schichten, die der Polylactidschicht benachbart sind, einen Brechungsindex aufweisen, der sich von dem Brechungsindex der Polylactidschichten um mindestens 0,03 unterscheidet.

2. Folie nach Anspruch 1, worin die aneinandergrenzenden Schichten, die der Polylactidschicht benachbart sinn, aus einem Polyester bestehen, der sich von dem Polylactid unterscheidet.

3. Folie nach Anspruch 1 oder 2, worin die aneinandergrenzenden Schichten, die der Polylactidschicht benachbart sind, aus einem Polyester bestehen, der einen höheren Brechungsindex aufweist als das Polylactid.

4. Folie nach einem der Ansprüche 1 bis 3, worin die aneinandergrenzenden Schichten, die der Polylactidschicht benachbart sind, aus einem Terephthalat-Polyester bestehen.

5. Folie nach Anspruch 4, worin es sich bei dem Terephthalat-Polyester um ein Polyethylenterephthalatglykol handelt.

6. Folie nach Anspruch 4, worin es sich bei dem Terephthalat-Polyester um ein Polyethylenterephthalat handelt.

7. Folie nach Anspruch 4, worin es sich bei dem Terephthalat-Polyester um ein Polyethylenterephthalat handelt.

8. Folie nach einem der Ansprüche 1 bis 7 mit mindestens 35 Schichten.

9. Folie nach einem der Ansprüche 1 bis 8, worin das Polylactid mehr als 93% L-Milchsäure-Einheiten umfaßt.

10. Folie nach einem der Ansprüche 1 bis 9, worin das Polylactid Poly-(D-lactid)- oder Poly-(L-lactid)-Homopolymere und Copolymere mit hohem D- oder L-Gehalt umfaßt.

## Revendications

1. Film comprenant une pluralité de couches contiguës étant essentiellement parallèles les unes aux autres, dans lequel au moins une couche sur deux parmi lesdites couches contiguës constituée de polylactide et lesdites couches contiguës qui sont adjacentes auxdites couches de polylactide ont un indice de réfraction différent d'au moins 0,03 par rapport à l'indice de réfraction desdites couches de polylactide.

2. Film selon la revendication 1, dans lequel lesdites couches contiguës qui sont adjacentes auxdites couches de polylactide sont constituées d'un polyester différent dudit polylactide.

3. Film selon la revendication 1 ou 2, dans lequel lesdites couches contiguës qui sont adjacentes auxdites couches de polylactide sont constituées d'un polyester ayant un indice de réfraction supérieur à l'indice de réfraction dudit polylactide.

4. Film selon l'une quelconque des revendications 1 à 3, dans lequel lesdites couches contiguës qui sont adjacentes auxdites couches de polylactide sont constituées de polyester de téréphtalate.

5. Film selon la revendication 4, dans lequel ledit polyester de téréphtalate est un téréphtalate de polyéthylène glycol.

6. Film selon la revendication 4, dans lequel ledit polyester de téréphtalate est un téréphtalate de polyéthylène.

7. Film selon la revendication 4, dans lequel le polyester de téréphtalate est un téréphtalate de polyéthylène.

8. Film selon l'une quelconque des revendications 1 à 7, ayant au moins 35 couches.

9. Film selon l'une quelconque des revendications 1 à 8, dans lequel ledit polylactide comprend plus de 93% de motifs d'acide L-lactique.

10. Film selon l'une quelconque des revendications 1 à 9, dans lequel ledit polylactide comprend des homopolymères de poly(D-lactide) ou de poly(L-lactide) et des copolymères à haute teneur en D ou L.
